# EUROPEAN PATENT SPECIFICATION

(11) **EP 2 046 383 B1**
(45) Date of publication and mention of the grant of the patent: **19.11.2014**
(21) Application number: 07764469.8
(22) Date of filing: 04.07.2007
(51) Int. Cl.: A61K 39/395, A61P 11/00, C07K 16/28

(54) **CD20 BINDING MOLECULES FOR THE TREATMENT OF COPD**
CD20-BINDUNGSMOLEKÜLE ZUR BEHANDLUNG VON COPD
MOLÉCULES DE LIAISON À CD20 POUR LE TRAITEMENT DE COPD

(30) Priority: 04.07.2006 DK 200600916; 05.07.2006 US 818593 P
(43) Date of publication of application: 15.04.2009
(73) Proprietor: Genmab A/S, 1260 Copenhagen K (DK)
(72) Inventor: PARREN, Paul, 3984 PR Odijk (NL); LISBY, Steen, 2000 Frederiksberg (DK); BAADSGAARD, Ole, 2900 Hellerup (DK)
(74) Representative: Genmab A/S
(86) International application number: PCT/DK2007/000339
(87) International publication number: WO 2008/003319

(56) References cited:
- WO-A-2005/040796
- WO-A2-2004/035607
- TEELING JESSICA L ET AL: "The biological activity of human CD20 monoclonal antibodies is linked to unique epitopes on CD20." JOURNAL OF IMMUNOLOGY (BALTIMORE, MD. : 1950) 1 JUL 2006, vol. 177, no. 1, 1 July 2006 (2006-07-01), pages 362-371, XP002453762 ISSN: 0022-1767
- TEELING J L ET AL: "Characterization of new human CD20 monoclonal antibodies with potent cytolytic activity against non-Hodgkin lymphomas" BLOOD, W.B.SAUNDERS COMPANY, ORLANDO, FL, US, vol. 104, no. 6, 15 September 2004 (2004-09-15), pages 1793-1800, XP002330734 ISSN: 0006-4971
- MAHLER DONALD A ET AL: "Efficacy and safety of a monoclonal antibody recognizing interleukin-8 in COPD: a pilot study." CHEST SEP 2004, vol. 126, no. 3, September 2004 (2004-09), pages 926-934, XP002453763 ISSN: 0012-3692

## Description

### FIELD OF THE INVENTION

The present invention relates to the use of a CD20 binding full-length antibody or bivalent fragment thereof comprising two Fab fragments linked by a disulfide bridge at the hinge region for the preparation of a medicament for the treatment of chronic obstructive pulmonary disease (COPD).

### BACKGROUND OF THE INVENTION

Chronic obstructive pulmonary disease (COPD) is a disease state characterized by airflow limitation that is not fully reversible. The airflow limitation is usually progressive and associated with an abnormal inflammatory response of the lungs to noxious particles or gases.

The prevalence of COPD has been estimated based on spirometry in two studies and range from 9.1% in people aged 40-69 years to 9.9% in people aged 60-74 years, cf. Dickinson JA et al. (1999) Thorax 54(6):501-505 and Pena VS et al. (2000) Chest 118(4):981-989.

Due to the asymptomatic nature of the disease in the early stage it is estimated that only about 20-50% of the patients are diagnosed. COPD is the fourth leading cause of death worldwide and the prevalence in the US is about 10 million people. The prevalence of COPD is increasing and it is predicted that it will become the third most common cause of death and the fifth most common cause of disability in the world in 2020, cf. Lopez (1998) Nat Med 4:1241-1243. COPD costs are increasing in the US and direct and indirect costs have been estimated to 30 billion US dollars, cf. Chen JC (1999) Cur Opinion Pulm Med 5:93-99.

The main etiology of COPD is cigarette smoking which accounts for approximately 80% of the cases. However, other genetic and/or environmental factors appear critical in that only 20% of smokers develop COPD. Despite smoke cessation (90% of COPD patients are ex-smokers) the disease continues to progress following initial onset, cf. Hogg JC et al. (2004) N Engl J Med 350(25):2645-2653. The chronic airflow limitation is caused by an abnormal inflammatory response in the small airways and lung parenchyma.

The mechanism responsible for the continued inflammation and progression of COPD in persons who stopped smoking is unknown. Cigarette smoking induces activation of the innate immune response. However, activation of the adaptive immune system may be responsible for the continuous inflammation despite of smoke cessation.

The innate immune system reacts rapidly but lacks memory. The innate immune system in the lungs includes the mucucilliary clearance system, epithelial barrier, macrophages and neutrophile granulocytes. The adaptive immune system reacts more slowly and expresses memory. The adaptive immune system in the lungs includes B-cells and T-cells comprising both humoral and cell mediated responses.

The lung inflammation is characterized by influx of innate inflammatory cells including macrophages, neutrophile granulocytes and adaptive inflammatory cells, including T-lymphocytes, especially CD8+ T-lymphocytes and CD20+ B-cells. The immune cells mediate the release of several pro-inflammatory and inflammatory mediators, including IL-8, TNF-alpha (TNF-α), IL-6, LTB4, TGF-beta (TGF-β), MCP-1 and proteases.

The inflammation results in chronic obstructive bronchitis, increased mucus production, plugging and destruction of alveoli resulting in emphysema. Fibrosis is also a prominent feature and may be one of the most important factors causing the increased airway resistance. Only cessation of smoking may decrease the decline of lung function and even after cessation of smoking the inflammation continues for years resulting in decreasing lung function.

Acute exacerbations of the disease are common and result in further decline of the lung function. During exacerbation there is an increase of IL-8, LTB4, neutrophils and IL-6 in the sputum. The exacerbations seem to be due to an enhanced inflammatory response and may be initiated by bacterial and viral infections.

COPD patients progress through several stages characterized by increasing symptoms and decreased lung function. The Global initiative for Chronic Obstructive Lung Disease (GOLD) has introduced a five-stage classification of the severity of COPD as follows:
**Stage 0:** chronic cough and sputum production, normal spirometry.
**Stage 1 :** FEV₁/FVC < 70% but FEV₁ ≥ 80%, usually chronic cough and sputum production.
**Stage 2:** 50% ≤ FEV₁ < 80%, shortness of breath on exertion.
**Stage 3:** 30% ≤ FEV₁ < 50% predicted, increased shortness of breath, repeated exacerbations.
**Stage 4:** FEV₁ < 30%, quality of life appreciably impaired, exacerbations may be life-threatening.

FEV₁ is forced expiratory volume in 1 second, and FVC if forced vital capacity. Assessment thereof may be performed by spirometry according to ERS (European Respiratory Society) / ATS (American Thoracic Society) standards.

O'Shaugnessy et al. (1997) Am J Respir Crit Care Med 155:852-857 disclose a study including 29 individuals where the authors did not find a difference in the number of B-cells in bronchial biopsies of large airways between patients with COPD and healthy non-smoking controls.

D'hulst AI et al. (2005) Respiratory Research 6:147 disclose a study wherein pulmonary emphysema is induced in SCID mice lacking lymphoid follicles as well as functional B- and T-cells with cigarette smoke-exposure (a murine model of COPD). The authors conclude that the adaptive immune system is not required per se to develop pulmonary emphysema in response to cigarette smoke-exposure, and that these results suggest that the innate immune system plays a major role in the pathogenesis of COPD and pulmonary emphysema.

Hogg JC et al. (2004) N Eng J Med 350:2645-2653 investigated the small airways in surgically resected lung tissue from 159 patients with COPD GOLD stages 0-4. The number of airways which contained neutrophils, macrophages, CD4+ cells, CD8+ cells, B-cells, and lymphoid follicles increased with disease progression, however the total accumulated volume of cells only increased for B-cells and CD8+ cells. Furthermore, there was a strong correlation between the progression of COPD and the percentage of airways with B-cell follicles. The B-cell follicles were surrounded by CD4+ cells.

Gosman MME et al. (2006) Eur Respir J 27:60-64 investigated central airways including bronchial biopsies from 114 patients with COPD GOLD stages 2 and 3 and 28 controls without COPD in the study. The study demonstrates an increased number of B-cells in the later stage compared to early stage patients. Despite discontinuation of smoking the inflammation and accelerated decline in lung function continued for many years.

WO 2004/035607 (Genmab A/S) discloses fully human CD20 monoclonal antibodies, including ofatumumab, as well as use thereof in the treatment of various diseases, including B cell lymphomas. No mention or suggestion is made that the antibodies may be useful for treating COPD.

Mahler Donald E et al: "Efficacy and safety of a monoclonal antibody recognizing interleukin-8 in COPD: a pilot study." Chest Sep 2004, vol. 126, no. 3, September 2004, pages 926-934, reports a pilot study investigating the use of an IL-8 antibody in COPD patients. The reference does not mention or suggest using CD20 as an alternative target to IL-8. In fact, the reference suggests targeting chemokines directly, describing that "a novel approach to reduce the inflammatory process in COPD is to target directly the chemokines that attract and activate neutrophils", cf. page 927, 1^{st} column, 1^{st} full paragraph thereof.

The molecular and cellular pathogenic mechanisms of COPD are not fully understood and there are many avenues to explore in order to provide new approaches for intervention through targeted therapy.

The main therapies of COPD include two classes of drugs: bronchodilators and anti-inflammatory agents. The main categories of bronchodilators include beta₂-agonists, methylxanthines and anticholinergics. The anti-inflammatory agents include inhaled corticosteroids and leukotriene antagonists. Inhaled corticosteroids may decrease the number of exacerbations and increase health status, but do not have any effect on the decline of lung function. Often bronchodilators and anti-inflammatory agents are used simultaneously. During acute exacerbation antibiotics may be added if a bacterial infection is suspected.

Cessation or abstinence from smoking may result in a partial inhibition of the decrease in lung function. However, there is no known cure at the present time and no pharmacological intervention has so far been able to affect the decline of lung function as measured by FEV₁.

Accordingly, there is still a need for an efficient therapy of COPD.

### SUMMARY OF THE INVENTION

The invention is based on the finding that CD20 binding molecules are efficient in treating COPD and/or related symptoms, such as bronchitis and emphysema.

Accordingly, the invention relates to use of a CD20 binding full-length antibody or bivalent fragment thereof comprising two Fab fragments linked by a disulfide bridge at the hinge region for the preparation of a medicament for the treatment of COPD. To a CD20 binding full-length antibody or bivalent fragment thereof comprising two Fab fragments linked by a disulfide bridge at the hinge region for use in the treatment of Chronic Obstructive Pulmonary Disease (COPD), as well as pharmaceutical compositions comprising a CD20 binding full-length antibody or bivalent fragment thereof comprising two Fab fragments linked by a disulfide bridge at the hinge region for use in the treatment of COPD.

Other features and advantages of the instant invention will be apparent from the following detailed description and examples which should not be construed as limiting.

### DEFINITIONS

The terms "CD20" and "CD20 antigen" are used interchangeably herein, and include any variants, isoforms and species homologs of human CD20, which are naturally expressed by cells or are expressed on cells transfected with the CD20 gene. Synonyms of CD20, as recognized in the art, include B-lymphocyte surface antigen B1, Leu-16 and Bp35. Human CD20 has UniProtKB/Swiss-Prot entry P11836.

The term "CD20 binding molecules" as used herein refer to any molecule that specifically binds to a portion of CD20 under cellular and/or physiological conditions for an amount of time sufficient to inhibit the activity of CD20 expressing cells and/or otherwise modulate a physiological effect associated with CD20; to allow detection by ELISA, western blot, or other similarly suitable binding technique described herein and/or known in the art and/or to otherwise be detectably bound thereto after a relevant period of time (for instance at least about 15 minutes, such as at least about 30 minutes, at least about 45 minutes, at least about 1 hour, at least about 2 hours, at least about 4 hours, at least about 6 hours, at least about 12 hours, such as about 1-24 hours, about 1-36 hours, about 1-48 hours, about 1-72 hours, about one week, or longer).

Binding molecules encompass, but are not limited to, antibodies and fragments thereof, peptides, and low molecular weight non-peptide molecules (also referred to as "small molecules") binding to the CD20 antigen.

The term "immunoglobulin" as used herein refers to a class of structurally related glycoproteins consisting of two pairs of polypeptide chains, one pair of light (L) low molecular weight chains and one pair of heavy (H) chains, all four inter-connected by disulfide bonds. The structure of immunoglobulins has been well characterized. See for instance Fundamental Immunology Ch. 7 (Paul, W., ed., 2nd ed. Raven Press, N.Y. (1989)). Briefly, each heavy chain typically is comprised of a heavy chain variable region (abbreviated herein as V_{H}) and a heavy chain constant region. The heavy chain constant region, C_{H}, typically is comprised of three domains, C_{H}1, C_{H}2, and C_{H}3. Each light chain typically is comprised of a light chain variable region (abbreviated herein as V_{L}) and a light chain constant region. The light chain constant region typically is comprised of one domain, C_{L}. The V_{H} and V_{L} regions may be further subdivided into regions of hypervariability (or hypervariable regions which may be hypervariable in sequence and/or form of structurally defined loops), also termed complementarity determining regions (CDRs), interspersed with regions that are more conserved, termed framework regions (FRs).

Each V_{H} and V_{L} is typically composed of three CDRs and four FRs, arranged from amino-terminus to carboxy-terminus in the following order: FR1, CDR1, FR2, CDR2, FR3, CDR3, FR4 (see also Chothia and Lesk J. Mol. Biol. 196, 901-917 (1987)). Typically, the numbering of amino acid residues in this region is performed by the method described in Kabat et al., Sequences of Proteins of Immunological Interest, 5th Ed. Public Health Service, National Institutes of Health, Bethesda, MD. (1991) (phrases, such as variable domain residue numbering as in Kabat or according to Kabat herein refer to this numbering system for heavy chain variable domains or light chain variable domains). Using this numbering system, the actual linear amino acid sequence of a peptide may contain fewer or additional amino acids corresponding to a shortening of, or insertion into, a FR or CDR of the variable domain. For example, a heavy chain variable domain may include a single amino acid insert (for instance residue 52a according to Kabat) after residue 52 of V_{H} CDR2 and inserted residues (for instance residues 82a, 82b, and 82c, etc. according to Kabat) after heavy chain FR residue 82. The Kabat numbering of residues may be determined for a given antibody by alignment at regions of homology of the sequence of the antibody with a "standard" Kabat numbered sequence.

The term "antibody" as used herein refers to an immunoglobulin molecule, a fragment of an immunoglobulin molecule, or a derivative of either thereof, which has the ability to specifically bind to an antigen under typical physiological conditions for a significant period of time, such as at least about 30 minutes, at least about 45 minutes, at least about one hour, at least about two hours, at least about four hours, at least about 8 hours, at least about 12 hours, about 24 hours or more, about 48 hours or more, about 3, 4, 5, 6, 7 or more days, etc., or any other relevant functionally-defined period (such as a time sufficient to induce, promote, enhance, and/or modulate a physiological response associated with antibody binding to the antigen and/or a time sufficient for the antibody to recruit an Fc-mediated effector activity).

The variable regions of the heavy and light chains of the immunoglobulin molecule contain a binding domain that interacts with an antigen. The constant regions of the antibodies may mediate the binding of the immunoglobulin to host tissues or factors, including various cells of the immune system (such as effector cells) and components of the complement system such as Clq, the first component in the classical pathway of complement activation.

The anti-CD20 antibody may be mono-, bi- or multispecific. Indeed, bispecific antibodies, diabodies, and the like, provided by the present invention may bind any suitable target in addition to a portion of CD20.

As indicated above, the term "antibody" as used herein, unless otherwise stated or clearly contradicted by the context, includes fragments of an antibody provided by any known technique, such as enzymatic cleavage, peptide synthesis and recombinant techniques that retain the ability to specifically bind to an antigen. It has been shown that the antigen-binding function of an antibody may be performed by fragments of a full-length (intact) antibody. Examples of antigen-binding fragments encompassed within the term "antibody" include, but are not limited to (i) a Fab fragment, a monovalent fragment consisting of the V_{L}, V_{H}, C_{L} and C_{H}1 domains; (ii) F(ab)₂ and F(ab')₂ fragments, bivalent fragments comprising two Fab fragments linked by a disulfide bridge at the hinge region; (iii) a Fd fragment consisting essentially of the V_{H} and C_{H}1 domains; (iv) a Fv fragment consisting essentially of the V_{L} and V_{H} domains of a single arm of an antibody, (v) a dAb fragment (Ward et al., Nature 341, 544-546 (1989)), which consists essentially of a V_{H} domain and also called domain antibodies (Holt et al. (November 2003) Trends Biotechnol. 21(11):484-90); (vi) a camelid antibody or nanobody (Revets et al. (January 2005) Expert Opin Biol Ther. 5(1):111-24), (vii) an isolated complementarity determining region (CDR), such as a V_{H} CDR3, (viii) a UniBody™, a monovalent antibody as disclosed in WO 2007/059782, (ix) a single chain antibody or single chain Fv (scFv), see for instance Bird et al., Science 242, 423-426 (1988) and Huston et al., PNAS USA 85, 5879-5883 (1988)), (x) a diabody (a scFv dimer), which can be monospecific or bispecific (see for instance PNAS USA 90(14), 6444-6448 (1993), EP 404097 or WO 93/11161 for a description of diabodies), a triabody or a tetrabody.

Although such fragments are generally included within the definition of an antibody, they collectively and each independently are unique features of the present invention, exhibiting different biological properties and utility. These and other useful antibody fragments in the context of the present invention are discussed further herein.

As used herein, "specific binding"' refers to the binding of a binding molecule, such as a full-length antibody or an antigen-binding fragment thereof, to a predetermined antigen. Typically, the antibody binds with an affinity corresponding to a K_{D} of about 10⁻⁷ M or less, such as about 10⁻⁸ M or less, such as about 10⁻⁹ M or less, about 10⁻¹⁰ M or less, or about 10⁻¹¹ M or even less, when measured for instance using sulfon plasmon resonance on BIAcore or as apparent affinities based on IC₅₀ values in FACS or ELISA, and binds to the predetermined antigen with an affinity corresponding to a K_{D} that is at least ten-fold lower, such as at least 100 fold lower, for instance at least 1000 fold lower, such as at least 10,000 fold lower, for instance at least 100,000 fold lower than its affinity for binding to a non-specific antigen (e.g., BSA, casein) other than the predetermined antigen or a closely-related antigen. When the K_{D} of the antigen binding peptide is very low (that is, the antigen binding peptide is highly specific), then the affinity for the antigen may be at least 10,000 or 100,000 fold lower than the affinity for a non-specific antigen.

It should be understood that the term antibody generally includes monoclonal antibodies as well as polyclonal antibodies. The antibodies can be human, humanized, chimeric, murine, etc. An antibody as generated can possess any isotype.

The term "human antibody", as used herein, is intended to include antibodies having variable and constant regions derived from human germline immunoglobulin sequences. The human antibodies of the present invention may include amino acid residues not encoded by human germline immunoglobulin sequences (for instance mutations introduced by random or site-specific mutagenesis *in vitro* or by somatic mutation *in vivo*). However, the term "human antibody", as used herein, is not intended to include antibodies in which CDR sequences derived from the germline of another mammalian species, such as a mouse, have been grafted into human framework sequences.

As used herein, a human antibody is "derived from" a particular germline sequence if the antibody is obtained from a system using human immunoglobulin sequences, for instance by immunizing a transgenic mouse carrying human immunoglobulin genes or by screening a human immunoglobulin gene library, and wherein the selected human antibody is at least 90%, such as at least 95%, for instance at least 96%, such as at least 97%, for instance at least 98%, or such as at least 99% identical in amino acid sequence to the amino acid sequence encoded by the germline immunoglobulin gene. Typically, a human antibody derived from a particular human germline sequence will display no more than 10 amino acid differences, such as no more than 5, for instance no more than 4, 3, 2, or 1 amino acid difference from the amino acid sequence encoded by the germline immunoglobulin gene. For V_{H} antibody sequences the V_{H} CDR3 domain is not included in such comparison.

The term "chimeric antibody" refers to an antibody that contains one or more regions from one antibody and one or more regions from one or more other antibodies. The term "chimeric antibody" includes monovalent, divalent, or polyvalent antibodies. A monovalent chimeric antibody is a dimer (HL)) formed by a chimeric H chain associated through disulfide bridges with a chimeric L chain. A divalent chimeric antibody is a tetramer (H₂L₂) formed by two HL dimers associated through at least one disulfide bridge. A polyvalent chimeric antibody may also be produced, for example, by employing a CH region that assembles into a molecule with 2+ binding sites (for instance from an IgM H chain, or µ chain). Typically, a chimeric antibody refers to an antibody in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see for instance US 4,816,567 and Morrison et al., PNAS USA 81, 6851-6855 (1984)). Chimeric antibodies are produced by recombinant processes well known in the art (see for instance Cabilly et al., PNAS USA 81, 3273-3277 (1984), Morrison et al., PNAS USA 81, 6851-6855 (1984), Boulianne et al., Nature 312, 643-646 (1984), EP125023, Neuberger et al., Nature 314, 268-270 (1985), EP171496, EP173494, WO 86/01533, EP184187, Sahagan et al., J. Immunol. 137, 1066-1074 (1986), WO 87/02671, Liu et al., PNAS USA 84, 3439-3443 (1987), Sun et al., PNAS USA 84, 214-218 (1987), Better et al., Science 240, 1041-1043 (1988) and Harlow et al., Antibodies: A Laboratory Manual, Cold Spring Harbor Laboratory Press, Cold Spring Harbor, N.Y., (1988)).

The term "humanized antibody" refers to a human antibody which contain minimal sequences derived from a non-human antibody. Typically, humanized antibodies are human immunoglobulins (recipient antibody) in which residues from a hypervariable region of the recipient are replaced by residues from a hypervariable region of a non-human species (donor antibody), such as mouse, rat, rabbit or non-human primate having the desired specificity, affinity, and capacity.

Furthermore, humanized antibodies may comprise residues which are not found in the recipient antibody or in the donor antibody. These modifications are made to further refine antibody performance. In general, a humanized antibody will comprise substantially all of at least one, and typically two, variable domains, in which all or substantially all of the hypervariable loops correspond to those of a non-human immunoglobulin and all or substantially all of the FR regions are those of a human immunoglobulin sequence. A humanized antibody optionally also will comprise at least a portion of a human immunoglobulin constant region. For further details, see Jones et al., Nature 321, 522-525 (1986), Riechmann et al., Nature 332, 323-329 (1988) and Presta, Curr. Op. Struct. Biol. 2, 593-596 (1992).

The terms "monoclonal antibody" or "monoclonal antibody composition" as used herein refer to a preparation of antibody molecules of single molecular composition. A monoclonal antibody composition displays a single binding specificity and affinity for a particular epitope. Accordingly, the term "human monoclonal antibody" refers to antibodies displaying a single binding specificity which have variable and constant regions derived from human germline immunoglobulin sequences. The human monoclonal antibodies may be generated by a hybridoma which includes a B cell obtained from a transgenic or transchromosomal nonhuman animal, such as a transgenic mouse, having a genome comprising a human heavy chain transgene and a light chain transgene, fused to an immortalized cell.

The term "recombinant human antibody", as used herein, includes all human antibodies that are prepared, expressed, created or isolated by recombinant means, such as (a) antibodies isolated from an animal (such as a mouse) that is transgenic or transchromosomal for human immunoglobulin genes or a hybridoma prepared therefrom (described further elsewhere herein), (b) antibodies isolated from a host cell transformed to express the antibody, such as from a transfectoma, (c) antibodies isolated from a recombinant, combinatorial human antibody library, and (d) antibodies prepared, expressed, created or isolated by any other means that involve splicing of human immunoglobulin gene sequences to other DNA sequences. Such recombinant human antibodies have variable and constant regions derived from human germline immunoglobulin sequences. In certain embodiments, however, such recombinant human antibodies may be subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

The terms "transgenic, non-human animal" refers to a non-human animal having a genome comprising one or more human heavy and/or light chain transgenes or transchromosomes (either integrated or non-integrated into the animal's natural genomic DNA) and which is capable of expressing fully human antibodies. For example, a transgenic mouse can have a human light chain transgene and either a human heavy chain transgene or human heavy chain transchromosome, such that the mouse produces human anti-CD20 antibodies when immunized with CD20 antigen and/or cells expressing CD20. The human heavy chain transgene may be integrated into the chromosomal DNA of the mouse, as is the case for transgenic mice, for instance the HuMAb-Mouse®, such as HCo7 or HCo12 mice, or the human heavy chain transgene may be maintained extrachromosomally, as is the case for the transchromosomal KM-Mouse® as described in WO 02/43478. Such transgenic and transchromosomal mice (collectively referred to herein as "transgenic mice") are capable of producing multiple isotypes of human monoclonal antibodies to a given antigen (such as IgG, IgA, IgM, IgD and/or IgE) by undergoing V-D-J recombination and isotype switching. Transgenic, nonhuman animals can also be used for production of antibodies against a specific antigen by introducing genes encoding such specific antibody, for example by operatively linking the genes to a gene which is expressed in the milk of the animal.

The term "treatment" as used herein means the administration of an effective amount of a therapeutically active compound of the present invention with the purpose of easing, ameliorating, arresting, or eradicating (curing) symptoms or disease states.

### SEQUENCE LISTING

| | | |
|---|---|---|
| SEQ ID NO:1 | 2F2 V_{H} | EVQLVESGGGLVQPGRSLRLSCAASGFTFNDYAMHWVR QAPGKGLEWVSTISWNSGSIGYADSVKGRFTISRDNAK KSLYLQMNSLRAEDTALYYCAKDIQYGNYYYGMDVWGQ GTTVTVSS |
| SEQ ID NO:2 | 2F2 V_{L} | EIVLTQSPATLSLSPGERATLSCRASQSVSSYLAWYQQK PGQAPRLLIYDASNRATGIPARFSGSGSGTDFTLTISSLE PEDFAVYYCQQRSNWPITFGQGTRLEIK |
| SEQ ID NO:3 | 2F2 V_{H} CDR1 | DYAMH |
| SEQ ID NO:4 | 2F2 V_{H} CDR2 | TISWNSGSIGYADSVKG |
| SEQ ID NO:5 | 2F2 V_{H} CDR3 | DIQYGNYYYGMDV |
| SEQ ID NO:6 | 2F2 V_{L} CDR1 | RASQSVSSYLA |
| SEQ ID NO:7 | 2F2 V_{L} CDR2 | DASNRAT |
| SEQ ID NO:8 | 2F2 V_{L} CDR3 | QQRSNWPIT |
| SEQ ID NO:9 | 11B8 V_{H} CDR3 | DYYGAGSFYDGLYGMDV |
| SEQ ID NO:10 | 2F2 V_{H} CDR1-CDR3 | DYAMHWVRQAPGKGLEWVSTISWNSGSIGYADSVKGR FTISRDNAKKSLYLQMNSLRAEDTALYYCAKDIQYGNYY YGMDV |
| SEQ ID NO:11 | 2C6 V_{H} CDR3 | DNQYGSGSTYGLGV |
| SEQ ID NO:12 | Human V_{H} DP-44/D3-10/JH6b germline sequence | EVQLVQSGGGLVHPGGSLRLSCAGSGFTFSSYAMHWVR QAPGKGLEWVSAIGTGGGTYYADSVKGRFTISRDNAKN SLYLQMNSLRAEDMAVYYCARDYYGSGSYYYYYYGMDV WGQGTTVTVSS |
| SEQ ID NO:13 | Human V_{L} L6/JK4 germline sequence | EIVLTQSPATLSLSPGERATLSCRASQSVSSYLAWYQQK PGQAPRLLIYDASNRATGIPARFSGSGSGTDFTLTISSLE PEDFAVYYCQQRSNWPLTFGGGTKVEIK |
| SEQ ID NO:14 | Human V_{H} 3-09/D4-11/JH6b germline sequence | EVQLVESGGGLVQPGRSLRLSCAASGFTFDDYAMHWVR QAPGKGLEWVSGISWNSGSIGYADSVKGRFTISRDNAK NSLYLQMNSLRAEDTALYYCAKDIDYYYYYYGMDVWGQ GTTVTVSS |
| SEQ ID NO:15 | Human V_{L} L6/JK5 germline sequence | EIVLTQSPATLSLSPGERATLSCRASQSVSSYLAWYQQK PGQAPRLLIYDASNRATGIPARFSGSGSGTDFTLTISSLE PEDFAVYYCQQRSNWPITFGQGTRLEIK |

### DETAILED DESCRIPTION OF THE INVENTION

In one aspect the invention relates to the use of a CD20 binding full-length antibody or bivalent fragment thereof comprising two Fab fragments linked by a disulfide bridge at the hinge region for the preparation of a medicament for the treatment of Chronic Obstructive Pulmonary Disease (COPD).

Treatment of COPD may result in the preparation of a medicament for the improvement of the lung function as measured by FEV₁/FVC (%) or FEV₁ (%) in a patient suffering from Chronic Obstructive Pulmonary Disease (COPD).

Treatment of COPD may result in the reduction of dyspnea as measured by the baseline dyspnea index (BDI) or the transitional dyspnea index (TDI) in a patient suffering from Chronic Obstructive Pulmonary Disease (COPD).

Treatment of COPD may result in the reduction of the incidence and/or severity of exacerbations in a patient suffering from Chronic Obstructive Pulmonary Disease (COPD).

Treatment of COPD may result in the reduction of inflammation in a patient suffering from Chronic Obstructive Pulmonary Disease (COPD) as measured by
a decrease in the induced sputum of the patient of one or more of the parameters: IL-4, IL-6, IL-8, TNF-α and GRO-α,
a decrease in the bronchoalveolar lavage (BAL) of the patient of one or more of the parameters: IL-4, IL-6, IL-8, TNF-α and GRO-α,
a decrease in the serum of the patient of one or more of the parameters IL-4, IL-6, IL-8, TNF-α and GRO-α, or
a decrease in a bronchial biopsy of the patient of one or more of the inflammatory cells: CD45+ cells, CD3+ cells, CD4+ cells, CD8+ cells, CD19+ cells, CD20+ cells, CD68+ cells, elastase, EG2 (eosinophils) and AA1 (mast cells).

In one embodiment of the invention, the medicament is suitable for intravenous, intraperitoneal, inhalation, intrabronchial, intraalveolar, intramuscular, subcutaneous or oral administration, such as for intravenous injection or infusion.

In one embodiment of the invention, the medicament is suitable for administration of the CD20 binding full-length antibody or bivalent fragment thereof comprising two Fab fragments linked by a disulfide bridge at the hinge region in an amount of from 10-2000 mg or in an amount of from 0.05-15 micromoles.

In one embodiment of the invention, the medicament is suitable for administration of the CD20 binding full-length antibody or bivalent fragment thereof comprising two Fab fragments linked by a disulfide bridge at the hinge region in a treatment regimen comprising administering 2 dosages of the CD20 binding full-length antibody or bivalent fragment thereof comprising two Fab fragments linked by a disulfide bridge at the hinge region each in an amount of from 100-2000 mg, 2 weeks apart.

In one embodiment of the invention, the medicament is suitable for administration of the CD20 binding full-length antibody or bivalent fragment thereof comprising two Fab fragments linked by a disulfide bridge at the hinge region in a treatment regimen comprising administering 1 dosage of CD20 binding full-length antibody or bivalent fragment thereof comprising two Fab fragments linked by a disulfide bridge at the hinge region in an amount of 100 mg, followed by 2 dosages of the CD20 binding full-length antibody or bivalent fragment thereof comprising two Fab fragments linked by a disulfide bridge at the hinge region each in an amount of from 300-2000 mg, which dosages are administered 1 and 3 weeks after the first dosage, respectively.

In one embodiment of the invention, the medicament is suitable for administration of the CD20 binding full-length antibody or bivalent fragment thereof comprising two Fab fragments linked by a disulfide bridge at the hinge region in a treatment regimen comprising administering a first dosage of the CD20 binding full-length antibody or bivalent fragment thereof comprising two Fab fragments linked by a disulfide bridge at the hinge region in an amount of 5-50 mg at day 0, a second dosage of the CD20 binding full-length antibody or bivalent fragment thereof comprising two Fab fragments linked by a disulfide bridge at the hinge region in an amount of 50-150 mg at day 1, a third dosage of the CD20 binding full-length antibody or bivalent fragment thereof comprising two Fab fragments linked by a disulfide bridge at the hinge region in an amount of 300-2000 mg at day 7, and a fourth dosage of the CD20 binding full-length antibody or bivalent fragment thereof comprising two Fab fragments linked by a disulfide bridge at the hinge region in an amount of 300-2000 mg at day 14 or day 21.

In one embodiment of the invention, the medicament is suitable for administration of the CD20 binding full-length antibody or bivalent fragment thereof comprising two Fab fragments linked by a disulfide bridge at the hinge region in a treatment regimen comprising administering a first dosage of the CD20 binding full-length antibody or bivalent fragment thereof comprising two Fab fragments linked by a disulfide bridge at the hinge region in an amount of 10 mg at day 0, a second dosage of the CD20 binding full-length antibody or bivalent fragment thereof comprising two Fab fragments linked by a disulfide bridge at the hinge region in an amount of 90 mg at day 1, a third dosage of the CD20 binding full-length antibody or bivalent fragment thereof comprising two Fab fragments linked by a disulfide bridge at the hinge region in an amount of 1000 mg at day 7, and a foruth dosage of the CD20 binding full-length antibody or bivalent fragment thereof comprising two Fab fragments linked by a disulfide bridge at the hinge region in an amount of 1000 mg at day 21.

Such treatment regimens may be repeated one or more times with an interval of 3-12 months, such as with an interval of 6 months.

In one embodiment the patients are pre-medicated prior to treatment with CD20 binding full-length antibody or bivalent fragment thereof comprising two Fab fragments linked by a disulfide bridge at the hinge region, such as for example with corticosteroids and antihistamines.

In one embodiment of the invention, the CD20 binding full-length antibody or bivalent fragment thereof comprising two Fab fragments linked by a disulfide bridge at the hinge region is a humanized polyclonal anti-CD20 antibody. Such antibodies may be generated in the transgenic rabbit disclosed in US 2003/0017534.

In one embodiment of the invention, the CD20 binding full-length antibody or bivalent fragment thereof comprising two Fab fragments linked by a disulfide bridge at the hinge region is an antibody against CD20, such a monoclonal antibody against CD20, such as a chimeric, humanized or human monoclonal antibody agaist CD20, preferably a human monoclonal antibody against CD20.

In one embodiment of the invention, the antibody against CD20 is a full-length antibody selected from the group consisting of a full-length IgG1 antibody, a full-length IgG2 antibody, a full-length IgG3 antibody, a full-length IgG4 antibody, a full-length IgM antibody, a full-length IgA1 antibody, a full-length IgA2 antibody, a full-length secretory IgA antibody, a full-length IgD antibody, and a full-length IgE antibody, wherein the antibody is glycosylated in a eukaryotic cell.

In one embodiment of the invention, the antibody is a full-length antibody, such as a full-length IgG1 antibody.

In one embodiment of the invention, the antibody against CD20 binds to mutant P172S CD20 (proline at position 172 mutated to serine) with at least the same affinity as to human CD20.

In one embodiment of the invention, the antibody against CD20 binds to an epitope on CD20
(i) which does not comprise or require the amino acid residue proline at position 172;
(ii) which does not comprise or require the amino acid residues alanine at position 170 or proline at position 172;
(iii) which comprises or requires the amino acid residues asparagine at position 163 and asparagine at position 166;
(iv) which does not comprise or require the amino acid residue proline at position 172, but which comprises or requires the amino acid residues asparagine at position 163 and asparagine at position 166; or
(v) which does not comprise or require the amino acid residues alanine at position 170 or proline at position 172, but which comprises or requires the amino acid residues asparagine at position 163 and asparagine at position 166.

In one embodiment of the invention, the antibody against CD20 binds to an epitope in the small first extracellular loop of human CD20.

In one embodiment of the invention, the antibody against CD20 binds to a discontinuous epitope on CD20.

In one embodiment of the invention, the antibody against CD20 binds to a discontinuous epitope on CD20, wherein the epitope comprises part of the first small extracellular loop and part of the second extracellular loop.

In one embodiment of the invention, the antibody against CD20 binds to a discontinuous epitope on CD20, wherein the epitope has residues AGIYAP of the small first extracellular loop and residues MESLNFIRAHTPYI of the second extracellular loop.

In one embodiment of the invention, the antibody against CD20 has one or more of the characteristics selected from the group consisting of:
(i) capable of inducing complement dependent cytotoxicity (CDC) of cells expressing CD20 in the presence of complement;
(ii) capable of inducing complement dependent cytotoxicity (CDC) of cells expressing CD20 and high levels of CD55 and/or CD59 in the presence of complement;
(iii) capable of inducing apoptosis of cells expressing CD20;
(iv) capable of inducing antibody dependent cellular cytotoxicity (ADCC) of cells expressing CD20 in the presence of effector cells;
(v) capable of inducing homotypic adhesion of cells which express CD20;
(vi) capable of translocating into lipid rafts upon binding to CD20;
(vii) capable of depleting cells expressing CD20;
(viii) capable of depleting cells expressing low levels of CD20 (CD20^{low} cells);
(ix) capable of effectively depleting B cells in situ in human tissues.

In one embodiment of the invention, the antibody against CD20 comprises a V_{H} CDR3 sequence selected from SEQ ID NOs: 5, 9, and 11.

In one embodiment of the invention, the antibody against CD20 comprises a V_{H} CDR1 of SEQ ID NO:3, a V_{H} CDR2 of SEQ ID NO:4, a V_{H} CDR3 of SEQ ID NO:5, a V_{L} CDR1 of SEQ ID NO:6, a V_{L} CDR2 of SEQ ID NO:7 and a V_{L} CDR3 sequence of SEQ ID NO:8.

In one embodiment of the invention, the antibody against CD20 comprises a V_{H} CDR1-CDR3 spanning sequence of SEQ ID NO:10.

In one embodiment of the invention, the antibody against CD20 has human heavy chain and human light chain variable regions comprising the amino acid sequences as set forth in SEQ ID NO:1 and SEQ ID NO:2, respectively; or amino acid sequences which are at least 95% homologous, and more preferably at least 98%, or at least 99% homologous to the amino acid sequences as set forth in SEQ ID NO:1 and SEQ ID NO:2, respectively.

In one embodiment of the invention the CD20 binding full-length antibody or bivalent fragment thereof comprising two Fab fragments linked by a disulfide bridge at the hinge region is selected from one of the anti-CD20 antibodies disclosed in WO 2004/035607, such as ofatumumab (2F2), 11B8, or 7D8, one of the antibodies disclosed in WO 2005/103081, such as 2C6, one of the antibodies disclosed in WO 2004/103404, AME-133 (humanized and optimized anti-CD20 monoclonal antibody, developed by Applied Molecular Evolution), one of the antibodies disclosed in US 2003/0118592, TRU-015 (CytoxB20G, a small modular immunopharma-ceutical fusion protein derived from key domains on an anti-CD20 antibody, developed by Trubion Pharmaceuticals Inc), one of the antibodies disclosed in WO 2003/68821, IMMU-106 (a humanized anti-CD20 monoclonal antibody), one of the antibodies disclosed in WO 2004/56312, ocrelizumab (2H7.v16, PRO-70769, R-1594), Bexxar® (tositumomab), and Rituxan® / MabThera® (rituximab).

In one embodiment of the invention, the antibody against CD20 is obtained by:
immunizing a transgenic non-human animal having a genome comprising a human heavy chain transgene or transchromosome and a human light chain transgene or transchromosome with a cell which has been transfected with human CD20, such that antibodies are produced by B cells of the animal;
isolating B cells of the animal;
fusing the B cells with myeloma cells to form immortal, hybridoma cells that secrete human monoclonal antibodies specific for human CD20; and
isolating the human monoclonal antibodies specific for human CD20 from the culture supernatant of the hybridoma, or the transfectoma derived from such hybridoma.

In one embodiment of the invention, the antibody against CD20 comprises a heavy chain variable region amino acid sequence derived from a human V_{H} DP-44/D3-10/JH6b germline sequence (SEQ ID NO:12) and a light chain variable region amino acid sequence derived from a human V_{L} L6/JK4 (SEQ ID NO:13) germline sequence; or a heavy chain variable region amino acid sequence derived from a human V_{H} 3-09/D4-11/JH6b germline sequence (SEQ ID NO:14) and a light chain variable region amino acid sequence derived from a human V_{L} L6/JK5 germline sequence (SEQ ID NO:15), wherein the human antibody specifically binds to CD20.

In one embodiment of the invention, the medicament is suitable for administration in combination with one or more further therapeutic agents, such as one or more further therapeutic agents selected from the group consisting of bronchodilators; anti-inflammatory agents; diuretics; digoxin; antihypertensives; cholesterol lowering drugs; anti-depressants; α₁-antitrypsin augmentation therapy (in patients with hereditary α₁-antitrypsin deficiency); mucolytic agents, such as ambroxolol, erdosteine, carbocysteine, and iodinated glycerol; antioxidants, such as N-acetylcysteine; immunostimulating agents; and antitussives.

In one embodiment of the invention the one or more further therapeutic agents are selected from the group consisting of bronchodilators and anti-inflammatory agents.

In one embodiment of the invention, the one or more further therapeutic agents comprise one or more bronchodilators selected from the group consisting of short acting or long acting beta₂-agonists, such as fenoterol, salbutamol, terbutaline, formoterol and salmeterol; methylxanthines, such as aminophylline and theophylline; anticholinergics; and inhaled anticholinergics, such as tiotropium, ipratropium and oxitropium.

In one embodiment of the invention, the one or more further therapeutic agents comprise one or more anti-inflammatory agents selected from the group consisting of inhaled corticosteroids; such as fluticazone, systemic corticosteroids, such as prednisolone; and leukotriene antagonists.

In one embodiment of the invention, the one or more further therapeutic agents are selected from the group consisting of anti-IL-8 antibodies, such as the anti-IL8 antibodies disclosed in WO 2004/058797, in particular 10F8, and the antibodies disclosed in WO 98/24893, in particular ABX-IL8, cf. Huang et al. (2002) Am. J. Pathol 161:125-134; anti-CD38 antibodies, such as the anti-CD38 antibodies disclosed in WO 2006/099875, anti-CD25 antibodies, such as Zenapax® (daclizumab), Simulect® (basiliximab) or the anti-CD25 antibodies disclosed in WO 2004/045512, in particular AB12, anti-CXCR1 antibodies, anti-CXCR2 antibodies, anti-CD8 antibodies and EGFr-inhibitors, such as anti-EGFr antibodies, for example the anti-EGFr antibodies disclosed in WO 2002/100348 or WO 2004/056847, in particular zalutumumab (2F8), cetuximab (Erbitux®), nimotuzumab (h-R3), panitumumab (ABX-EGF), and matuzumab (EMD72000).

Any suitable combination of further therapeutic agents may be used in combination with the CD20 binding full-length antibody or bivalent fragment thereof comprising two Fab fragments linked by a disulfide bridge at the hinge region. The CD20 binding full-length antibody or bivalent fragment thereof comprising two Fab fragments linked by a disulfide bridge at the hinge region may be administered simultaneously or sequentially with the further therapeutic agents in any order.

In one embodiment of the invention, the CD20 binding full-length antibody or bivalent fragment thereof comprising two Fab fragments linked by a disulfide bridge at the hinge region, such as ofatumumab, is administered in combination with salmeterol and fluticazone.

In another aspect the invention relates to a CD20 binding full-length antibody or bivalent fragment thereof comprising two Fab fragments linked by a disulfide bridge at the hinge region for use in the treatment of Chronic Obstructive Pulmonary Disease (COPD), as disclosed in any one of the above embodiments.

In another aspect the invention relates to a pharmaceutical composition containing a CD20 binding full-length antibody or bivalent fragment thereof comprising two Fab fragments linked by a disulfide bridge at the hinge region for use in the treatment of Chronic Obstructive Pulmonary Disease (COPD), as disclosed in any one of the above embodiments.

Methods of treating Chronic Obstructive Pulmonary Disease (COPD) in a patient comprise administering to the patient in need thereof a CD20 binding full-length antibody or bivalent fragment thereof comprising two Fab fragments linked by a disulfide bridge at the hinge region in an amount effective to treat the disease, as disclosed in any one of the above embodiments.

In accordance with the present invention, a CD20 binding full-length antibody or bivalent fragment thereof comprising two Fab fragments linked by a disulfide bridge at the hinge region can be administered to a patient suffering from COPD to improve the patient's condition. Accordingly, patients suffering from one or more of the various indications of COPD, such as chronic bronchitis, emphysema, irreversible asthma, bronchiectasis, immunoglobulin deficiency, and cystic fibrosis can be treated using a CD20 binding full-length antibody or bivalent fragment thereof comprising two Fab fragments linked by a disulfide bridge at the hinge region according to the present invention.

In accordance with the present invention, a CD20 binding full-length antibody or bivalent fragment thereof comprising two Fab fragments linked by a disulfide bridge at the hinge region can be administered to alleviate a patient's symptoms, or can be administered to counteract a mechanism of the disorder itself. It will be appreciated by those of skill in the art that these treatment purposes are often related and that treatments can be tailored for particular patients based on various factors. These factors can include the age, gender, or health of the patient, the progression of COPD, the degree of dyspnea, the amount of tissue damage to the patient's respiratory tract, the patient's smoking history, and various environmental factors (including, for example, temperature, humidity, and air pollution) which could contribute to the patient's condition. The treatment methodology for a patient, such as dosage, timing of administration, and route of administration, can be tailored accordingly and by concurrent or sequential administration of other therapies.

Actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, or the ester, salt or amide thereof, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required. For example, the physician or veterinarian could start doses of the compounds of the invention employed in the pharmaceutical composition at levels lower than that required in order to achieve the desired therapeutic effect and gradually increase the dosage until the desired effect is achieved. In general, a suitable daily dose of a composition of the invention will be that amount of the compound which is the lowest dose effective to produce a therapeutic effect. Such an effective dose will generally depend upon the factors described above. It is preferred that administration be intravenous, intramuscular, intraperitoneal, by inhalation or subcutaneous. If desired, the effective daily dose of a therapeutic composition may be administered as two, three, four, five, six or more sub-doses administered separately at appropriate intervals throughout the day, optionally, in unit dosage forms. While it is possible for a compound of the present invention to be administered alone, it is preferable to administer the compound as a pharmaceutical formulation (composition). The dosage can be determined or adjusted by measuring the amount of circulating CD20 binding full-length antibodies or bivalent fragments thereof comprising two Fab fragments linked by a disulfide bridge at the hinge region or presence of CD20 binding full-length antibodies or bivalent fragments thereof comprising two Fab fragments linked by a disulfide bridge at the hinge region in BAL or induced sputum at different time points following administration in a biological sample, e.g. by making use of anti-idiotypic antibodies targeting anti-CD20 antibodies in a method for detecting anti-CD20 antibodies, for instance in an ELISA set-up, or by using other specific methods to detect the anti-CD20 antibodies, for instance by an ELISA using CD20 as coating.

The CD20 binding full-length antibody or bivalent fragment thereof comprising two Fab fragments linked by a disulfide bridge at the hinge region may be administered to the patients in a dosage of from 100-2000 mg, such as 100 mg, 350 mg, 700 mg, 1000 mg, 1500 or 2000 mg.

The CD20 binding full-length antibody or bivalent fragment thereof comprising two Fab fragments linked by a disulfide bridge at the hinge region may beadministered to the patients in a dosage of from 0.5-15 micromoles, such as 0.5, 1, 2, 5, 7, 10 , 14 or 15 micromoles.

The CD20 binding full-length antibody or bivalent fragment thereof comprising two Fab fragments linked by a disulfide bridge at the hinge region may be administered by infusion in a dosage of 1.0 mg/kg, 1.5 mg/kg, 2.0 mg/kg, 4 mg/kg, 8 mg/kg, 14 mg/kg or 20 mg/kg.

The CD20 binding full-length antibody or bivalent fragment thereof comprising two Fab fragments linked by a disulfide bridge at the hinge region may be administered in a treatment regimen comprising administering 1-3 dosages of the CD20 binding full-length antibody or bivalent fragment thereof comprising two Fab fragments linked by a disulfide bridge at the hinge region in an amount of from 100-2000 mg, such as 350 mg, 700 mg, 1000 mg, 1500 mg or 2000 mg, 1-3 weeks apart, such as 2 dosages each in an amount of 1000 mg, 2 weeks apart.
The CD20 binding full-length antibody or bivalent fragment thereof comprising two Fab fragments linked by a disulfide bridge at the hinge region may be administered in a treatment regimen comprising administering 1 dosage of the CD20 binding full-length antibody or bivalent fragment thereof comprising two Fab fragments linked by a disulfide bridge at the hinge region in an amount of 100 mg, followed by 1-3 dosages of the CD20 binding full-length antibody or bivalent fragment thereof comprising two Fab fragments linked by a disulfide bridge at the hinge region in an amount of from 300-2000 mg, such as such as 350 mg, 700 mg, 1000 mg, 1500 mg or 2000 mg, which dosages are administered 1 and 2-3 weeks after the first dosage, respectively. For example, 100 mg is administered at day 0 followed by a 1000 mg dosage at day 7 and a 1000 mg dosage at day 21.

In one embodiment of the invention, the CD20 binding full-length antibody or bivalent fragment thereof comprising two Fab fragments linked by a disulfide bridge at the hinge region is administered in a treatment regimen comprising administering a first dosage of the CD20 binding full-length antibody or bivalent fragment thereof comprising two Fab fragments linked by a disulfide bridge at the hinge region in an amount of 5-50 mg at day 0, followed by a second 50-150 mg dosage at day 1, a third 300-2000 mg dosage at day 7 and a fourth 300-2000 mg dosage dosage at day 14 or 21. For example, 10 mg is administered at day 0, followed by a 100 mg dosage at day 1, a 1000 mg dosage at day 7, and a 1000 mg dosage at day 21.

These treatment regimens may be repeated one or more times as necessary with an interval of 3-12 months, such as with an interval of 3, 6, 9 or 12 months.

These treatment regiments may be administered in combination with one or more further drugs, such as one or more of the further drugs described above. In one embodiment the patients are pre-medicated prior to treatment with the CD20 binding full-length antibody or bivalent fragment thereof comprising two Fab fragments linked by a disulfide bridge at the hinge region, such as for example with corticosteroids and antihistamines.

Therapeutic compositions can be administered with medical devices known in the art. For example, in an embodiment, a pharmaceutical composition of the invention can be administered with a needleless hypodermic injection device, such as the devices disclosed in U.S. Patent Nos. 5,399,163; 5,383,851; 5,312,335; 5,064,413; 4,941,880; 4,790,824; or 4,596,556. Examples of well-known implants and modules useful in the present invention include: U.S. Patent No. 4,487,603, which discloses an implantable micro-infusion pump for dispensing medication at a controlled rate; U.S. Patent No. 4,486,194, which discloses a therapeutic device for administering medicaments through the skin; U.S. Patent No. 4,447,233, which discloses a medication infusion pump for delivering medication at a precise infusion rate; U.S. Patent No. 4,447,224, which discloses a variable flow implantable infusion apparatus for continuous drug delivery; U.S. Patent No. 4,439,196, which discloses an osmotic drug delivery system having multi-chamber compartments; and U.S. Patent No. 4,475,196, which discloses an osmotic drug delivery system. Many other such implants, delivery systems, and modules are known to those skilled in the art.

A "therapeutically effective dosage" for COPD preferably will result in a reduction in the overall COPD evaluation. This can, e.g., be evaluated by one of the tests disclosed in Example 3.

In one embodiment, the present invention provides a pharmaceutical composition comprising a therapeutically effective amount of a CD20 binding full-length antibody or bivalent fragment thereof comprising two Fab fragments linked by a disulfide bridge at the hinge region of the present invention. The pharmaceutical compositions may be formulated with pharmaceutically acceptable carriers or diluents as well as any other known adjuvants and excipients in accordance with conventional techniques, such as those disclosed in Remington: The Science and Practice of Pharmacy, 19th Edition, Gennaro, Ed., Mack Publishing Co., Easton, PA, 1995.

The pharmaceutically acceptable carriers or diluents as well as any other known adjuvants and excipients should be suitable for the chosen compound of the present invention and the chosen mode of administration. Suitability for carriers and other components of pharmaceutical compositions is determined based on the lack of significant negative impact on the desired biological properties of the chosen compound or pharmaceutical composition of the present invention (e.g., less than a substantial impact (10% or less relative inhibition, 5% or less relative inhibition, etc.) on antigen binding.

A pharmaceutical composition of the present invention may also include diluents, fillers, salts, buffers, detergents (e. g., a nonionic detergent, such as Tween-80), stabilizers, stabilizers (e. g., sugars or protein-free amino acids), preservatives, tissue fixatives, solubilizers, and/or other materials suitable for inclusion in a pharmaceutical composition.

The actual dosage levels of the active ingredients in the pharmaceutical compositions of the present invention may be varied so as to obtain an amount of the active ingredient which is effective to achieve the desired therapeutic response for a particular patient, composition, and mode of administration, without being toxic to the patient. The selected dosage level will depend upon a variety of pharmacokinetic factors including the activity of the particular compositions of the present invention employed, the route of administration, the time of administration, the rate of excretion of the particular compound being employed, the duration of the treatment, other drugs, compounds and/or materials used in combination with the particular compositions employed, the age, sex, weight, condition, general health and prior medical history of the patient being treated, and like factors well known in the medical arts.

The CD20 binding full-length antibody or bivalent fragment thereof comprising two Fab fragments linked by a disulfide bridge at the hinge region of the present invention may be administered via any suitable route, such as an oral, nasal, inhalable, intrabronchial, intraalveolar, topical (including buccal, transdermal and sublingual), rectal, vaginal and/or parenteral route

In one embodiment, a pharmaceutical composition of the present invention is administered parenterally.

The phrases "parenteral administration" and "administered parenterally" as used herein means modes of administration other than enteral and topical administration, usually by injection, and include epidermal, intravenous, intramuscular, intraarterial, intrathecal, intracapsular, intraorbital, intracardiac, intradermal, intraperitoneal, intratendinous, transtracheal, subcutaneous, subcuticular, intraarticular, subcapsular, subarachnoid, intraspinal, intracranial, intrathoracic, epidural and intrasternal injection and infusion.

In one embodiment the pharmaceutical composition is administered by intravenous or subcutaneous injection or infusion. For example the pharmaceutical composition may be administered over 2-8 hours, such as 4 hours, in order to reduce side effects.

In one embodiment the pharmaceutical composition is administered by inhalation. Fab fragments of anti-CD20 antibodies may be suitable for such administration route, cf. Crowe et al. (February 15, 1994) Proc Natl Acad Sci USA, 91(4):1386-1390.

In one embodiment the pharmaceutical composition is administered in crystalline form by subcutaneous injection, cf. Yang et al., PNAS USA 100(12), 6934-6939 (2003).

Regardless of the route of administration selected, the CD20 binding full-length antibody or bivalent fragment thereof comprising two Fab fragments linked by a disulfide bridge at the hinge region, which may be used in the form of a pharmaceutically acceptable salt or in a suitable hydrated form, is formulated into pharmaceutically acceptable dosage forms by conventional methods known to those of skill in the art. A "pharmaceutically acceptable salt" refers to a salt that retains the desired biological activity of the parent compound and does not impart any undesired toxicological effects (see for instance Berge, S.M. et al., J. Pharm. Sci. 66, 1-19 (1977)). Examples of such salts include acid addition salts and base addition salts. Acid addition salts include those derived from nontoxic inorganic acids, such as hydrochloric, nitric, phosphoric, sulfuric, hydrobromic, hydroiodic, phosphorous acids and the like, as well as from nontoxic organic acids, such as aliphatic mono- and dicarboxylic acids, phenyl-substituted alkanoic acids, hydroxy alkanoic acids, aromatic acids, aliphatic and aromatic sulfonic acids and the like. Base addition salts include those derived from alkaline earth metals, such as sodium, potassium, magnesium, calcium and the like, as well as from nontoxic organic amines, such as N,N'-dibenzylethylenediamine, N-methylglucamine, chloroprocaine, choline, diethanolamine, ethylenediamine, procaine and the like.

Pharmaceutically acceptable carriers include any and all suitable solvents, dispersion media, coatings, antibacterial and antifungal agents, isotonicity agents, antioxidants and absorption delaying agents, and the like that are physiologically compatible with a compound of the present invention.

Examples of suitable aqueous and nonaqueous carriers which may be employed in the pharmaceutical compositions of the present invention include water, saline, phosphate buffered saline, ethanol, dextrose, polyols (such as glycerol, propylene glycol, polyethylene glycol, and the like), and suitable mixtures thereof, vegetable oils, such as olive oil, corn oil, peanut oil, cottonseed oil, and sesame oil, carboxymethyl cellulose colloidal solutions, tragacanth gum and injectable organic esters, such as ethyl oleate, and/or various buffers. Other carriers are well known in the pharmaceutical arts.

Pharmaceutically acceptable carriers include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the present invention is contemplated.

Proper fluidity may be maintained, for example, by the use of coating materials, such as lecithin, by the maintenance of the required particle size in the case of dispersions, and by the use of surfactants.

Pharmaceutical compositions containing the CD20 binding full-length antibody or bivalent fragment thereof comprising two Fab fragments linked by a disulfide bridge at the hinge region may also comprise pharmaceutically acceptable antioxidants for instance (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Pharmaceutical compositions of the present invention may also comprise isotonicity agents, such as sugars, polyalcohols such as mannitol, sorbitol, glycerol or sodium chloride in the compositions.

Pharmaceutically acceptable diluents include saline and aqueous buffer solutions.

The pharmaceutical compositions containing the CD20 binding full-length antibody or bivalent fragment thereof comprising two Fab fragments linked by a disulfide bridge at the hinge region may also contain one or more adjuvants appropriate for the chosen route of administration, such as preservatives, wetting agents, emulsifying agents, dispersing agents, preservatives or buffers, which may enhance the shelf life or effectiveness of the pharmaceutical composition. Compounds of the present invention may for instance be admixed with lactose, sucrose, powders (e.g., starch powder), cellulose esters of alkanoic acids, stearic acid, talc, magnesium stearate, magnesium oxide, sodium and calcium salts of phosphoric and sulphuric acids, acacia, gelatin, sodium alginate, polyvinylpyrrolidine, and/or polyvinyl alcohol. Other examples of adjuvants are QS21, GM-CSF, SRL-172, histamine dihydrochloride, thymocartin, Tio-TEPA, monophosphoryl-lipid A/microbacteria compositions, alum, incomplete Freund's adjuvant, montanide ISA, ribi adjuvant system, TiterMax adjuvant, syntex adjuvant formulations, immune-stimulating complexes (ISCOMs), gerbu adjuvant, CpG oligodeoxynucleotides, lipopolysaccharide, and polyinosinic:polycytidylic acid.

Prevention of presence of microorganisms may be ensured both by sterilization procedures and by the inclusion of various antibacterial and antifungal agents, for example, paraben, chlorobutanol, phenol, sorbic acid, and the like. In addition, prolonged absorption of the injectable pharmaceutical form may be brought about by the inclusion of agents which delay absorption, such as aluminum monostearate and gelatin.

The pharmaceutical compositions containing a CD20 binding full-length antibody or bivalent fragment thereof comprising two Fab fragments linked by a disulfide bridge at the hinge region comprising a compound of the present invention may also include a suitable salt therefore. Any suitable salt, such as an alkaline earth metal salt in any suitable form (e.g., a buffer salt), may be used in the stabilization of the compound of the present invention. Suitable salts typically include sodium chloride, sodium succinate, sodium sulfate, potassium chloride, magnesium chloride, magnesium sulfate, and calcium chloride. In one embodiment, an aluminum salt is used to stabilize a compound of the present invention in a pharmaceutical composition of the present invention, which aluminum salt also may serve as an adjuvant when such a composition is administered to a patient.

The pharmaceutical compositions containing a CD20 binding full-length antibody or bivalent fragment thereof comprising two Fab fragments linked by a disulfide bridge at the hinge region may be in a variety of suitable forms. Such forms include, for example, liquid, semi-solid and solid dosage forms, such as liquid solutions (e.g., injectable and infusible solutions), dispersions or suspensions, emulsions, microemulsions, gels, creams, granules, powders, tablets, pills, powders, liposomes, dendrimers and other nanoparticles (see for instance Baek et al., Methods Enzymol. 362, 240-9 (2003), Nigavekar et al., Pharm Res. 21(3), 476-83 (2004), microparticles, and suppositories.

The optimal form depends on the mode of administration chosen and the nature of the composition. Formulations may include, for instance, powders, pastes, ointments, jellies, waxes, oils, lipids, lipid (cationic or anionic) containing vesicles, DNA conjugates, anhydrous absorption pastes, oil-in-water and water-in-oil emulsions, emulsions carbowax (polyethylene glycols of various molecular weights), semi-solid gels, and semi-solid mixtures containing carbowax. Any of the foregoing may be appropriate in treatments and therapies in accordance with the present invention, provided that the CD20 binding full-length antibody or bivalent fragment thereof comprising two Fab fragments linked by a disulfide bridge at the hinge region in the pharmaceutical composition is not inactivated by the formulation and the formulation is physiologically compatible and tolerable with the route of administration. See also for instance Powell et al., "Compendium of excipients for parenteral formulations" PDA J Pharm Sci Technol. 52, 238-311 (1998) and the citations therein for additional information related to excipients and carriers well known to pharmaceutical chemists.

The CD20 binding full-length antibody or bivalent fragment thereof comprising two Fab fragments linked by a disulfide bridge at the hinge region may be prepared with carriers that will protect the compound against rapid release, such as a controlled release formulation, including implants, transdermal patches, and microencapsulated delivery systems. Such carriers may include gelatin, glyceryl monostearate, glyceryl distearate, biodegradable, biocompatible polymers, such as ethylene vinyl acetate, polyanhydrides, polyglycolic acid, collagen, polyorthoesters, and polylactic acid alone or with a wax, or other materials well known in the art.. Methods for the preparation of such formulations are generally known to those skilled in the art. See e.g., Sustained and Controlled Release Drug Delivery Systems, J.R. Robinson, ed., Marcel Dekker, Inc., New York, 1978.

To administer the pharmaceutical compositions containing the CD20 binding full-length antibodies or bivalent fragments thereof comprising two Fab fragments linked by a disulfide bridge at the hinge region by certain routes of administration according to the invention, it may be necessary to coat the CD20 binding full-length antibody or bivalent fragment thereof comprising two Fab fragments linked by a disulfide bridge at the hinge region with, or co-administer the CD20 binding full-length antibody or bivalent fragment thereof comprising two Fab fragments linked by a disulfide bridge at the hinge region with, a material to prevent its inactivation. For example, the CD20 binding full-length antibody or bivalent fragment thereof comprising two Fab fragments linked by a disulfide bridge at the hinge region may be administered to a subject in an appropriate carrier, for example, liposomes, or a diluent. Liposomes include water-in-oil-in-water CGF emulsions as well as conventional liposomes (Strejan et al., J. Neuroimmunol. 7, 27 (1984)).

Depending on the route of administration, the CD20 binding full-length antibody or bivalent fragment thereof comprising two Fab fragments linked by a disulfide bridge at the hinge region may be coated in a material to protect the CD20 binding full-length antibody or bivalent fragment thereof comprising two Fab fragments linked by a disulfide bridge at the hinge region from the action of acids and other natural conditions that may inactivate the compound. For example, the CD20 binding full-length antibody or bivalent fragment thereof comprising two Fab fragments linked by a disulfide bridge at the hinge region may be administered to a subject in an appropriate carrier, for example, liposomes. Liposomes include water-in-oil-in-water CGF emulsions as well as conventional liposomes (Strejan et al., J. Neuroimmunol. 7, 27 (1984)).

Pharmaceutically acceptable carriers for parenteral administration include sterile aqueous solutions or dispersions and sterile powders for the extemporaneous preparation of sterile injectable solutions or dispersion. The use of such media and agents for pharmaceutically active substances is known in the art. Except insofar as any conventional media or agent is incompatible with the active compound, use thereof in the pharmaceutical compositions of the present invention is contemplated. Supplementary active compounds may also be incorporated into the compositions.

Pharmaceutical compositions for injection must typically be sterile and stable under the conditions of manufacture and storage. The composition may be formulated as a solution, microemulsion, liposome, or other ordered structure suitable to high drug concentration. The carrier may be a aqueous or nonaqueous solvent or dispersion medium containing for instance water, ethanol, polyols (such as glycerol, propylene glycol, polyethylene glycol, and the like), and suitable mixtures thereof, vegetable oils, such as olive oil, and injectable organic esters, such as ethyl oleate. The proper fluidity may be maintained, for example, by the use of a coating, such as lecithin, by the maintenance of the required particle size in the case of dispersion and by the use of surfactants. In many cases, it will be preferable to include isotonic agents, for example, sugars, polyalcohols, such as glycerol, mannitol, sorbitol, or sodium chloride in the composition. Prolonged absorption of the injectable compositions may be brought about by including in the composition an agent that delays absorption, for example, monostearate salts and gelatin. Sterile injectable solutions may be prepared by incorporating the active compound in the required amount in an appropriate solvent with one or a combination of ingredients e.g. as enumerated above, as required, followed by sterilization microfiltration.

Generally, dispersions are prepared by incorporating the active compound into a sterile vehicle that contains a basic dispersion medium and the required other ingredients e.g. from those enumerated above. In the case of sterile powders for the preparation of sterile injectable solutions, examples of methods of preparation are vacuum drying and freeze-drying (lyophilization) that yield a powder of the active ingredient plus any additional desired ingredient from a previously sterile-filtered solution thereof.

Sterile injectable solutions may be prepared by incorporating the active compound in the required amount in an appropriate solvent with one or a combination of ingredients enumerated above, as required, followed by sterilization microfiltration. Generally, dispersions are prepared by incorporating the active compound into a sterile vehicle that contains a basic dispersion medium and the required other ingredients from those enumerated above. In the case of sterile powders for the preparation of sterile injectable solutions, examples of methods of preparation are vacuum drying and freeze-drying (lyophilization) that yield a powder of the active ingredient plus any additional desired ingredient from a previously sterile-filtered solution thereof.

The pharmaceutical composition may contain a combination of multiple (e.g., two or more) CD20 binding full-length antibodies or bivalent fragments thereof comprising two Fab fragments linked by a disulfide bridge at the hinge region which act by different mechanisms, e.g., one anti-CD20 antibody which predominately acts by inducing CDC in combination with another anti-CD20 antibody which predominately acts by inducing apoptosis.

### EXAMPLES

The present invention is further illustrated by the following examples which should not be construed as further limiting.

### Example 1 : Formulation of ofatumumab (2F2)

The following 20 mg/ml aqueous formulation of ofatumumab is prepared by standard procedures:

| **Ingredient** | **Quantity per ml** | **Function** |
|---|---|---|
| Ofatumumab drug substance | 20 mg | Active ingredient |
| Sodium Citrate USP/EP | 8.549 mg | Buffering and stabilizing agent |
| Citric Acid USP/EP | 0.195 mg | Buffering and stabilizing agent |
| Sodium Chloride USP/EP | 5.844 mg | Isotonic agent |
| Water for injection USP/EP q.s. to | 1 ml | Solvent |

### Example 2: Treatment regimen with ofatumumab

In one embodiment of the invention patients with COPD, such as COPD in GOLD stage 1, 2, 3 or 4, are administered with 100 mg of ofatumumab by intravenous infusion over 2-8 hours, such as over 2-4 hours at day 0. This administration is followed by 2 dosages of 1000 mg of ofatumumab administered at day 7 and 21 day, respectively, by intravenous infusion over 2-8 hours, such as over 2-4 hours. Pre-medication may be given, for example as follows: 1 mg clemastine (or an equivalent dosage of another antihistamine) and 100 mg prednisolone (or an equivalent dosage of another glucocorticosteroid) are administered p.o. on the day prior to the first infusion of 10 mg ofatumumab, 2 mg clemastine and 100 mg prednisolone are administered i.v. 1 to 2 hours prior to the said first infusion of 10 mg ofatumumab, and 1 g paracetamol is administered p.o. 30 minutes to 2 hours prior to the said first infusion of 10 mg ofatumumab. Such pre-medication may be administered prior to each of the subsequent ofatumumab infusions.

The treatment regimen may be repeated one or more times every 3-12 months, such as every 6 months as necessary.

The patients may simultaneously be treated with other drugs, such as inhaled steroids and long-acting beta₂ agonists.

### Example 3: Treatment regimen with ofatumumab

In one embodiment of the invention patients with COPD, such as COPD in GOLD stage 1, 2, 3 or 4, are administered with 2 doses of 1000 mg of ofatumumab two weeks apart. The drug is administered by intravenous infusion over 2-8 hours, such as over 2-4 hours. Pre-medication such as disclosed in example 2 may be administered.

The treatment regimen may be repeated one or more times every 3-12 months, such as every 6 months as necessary.

The patients may simultaneously be treated with other drugs, such as inhaled steroids and long-acting beta₂ agonists.

### Example 4: Treatment regimen with ofatumumab

In one embodiment of the invention patients with COPD, such as COPD in GOLD stage 1, 2, 3 or 4, are administered with a first dosage of 10 mg of ofatumumab at day 0, a second dosage of 90 mg of ofatumumab at day 1, a third dosage of 1000 mg of ofatumumab at day 7, and a fourth dosage of 1000 mg of ofatumumab at day 21. The drug is administered by intravenous infusion over 2-8 hours, such as over 2-4 hours.

Pre-medication such as disclosed in example 2 may be administered.

The treatment regimen may be repeated one or more times every 3-12 months, such as every 6 months as necessary.

The patients may simultaneously be treated with other drugs, such as inhaled steroids and long-acting beta₂ agonists.

### Example 5: Efficacy of the CD20 binding full-length antibody or bivalent fragment thereof comprising two Fab fragments linked by a disulfide bridge at the hinge region in the treatment of COPD

The efficacy of the CD20 binding full-length antibody or bivalent fragment thereof comprising two Fab fragments linked by a disulfide bridge at the hinge region can be measured pursuant to one or more of the following tests:

### Induced sputum test

After pre-medication with 1.5 mg of terbutaline, administered in 3 doses via turbohaler, sputum is induced by inhalation with concentrated hypertonic saline via a nebulizer (Portaneb) for periods of 5 minutes for a total time of 15 minutes. For safety reasons the FEV₁ will be monitored every 5 minutes, followed by a dose of 0.5 mg terbutaline and the induction will be stopped if the FEV₁ falls with more than 20% from baseline.

The sputum samples are collected and analysed for the following parameters according to standard procedures:
IL-4, IL-6, IL-8, TNF-α, GRO-α, urea and albumin

Urea and albumin are included as reference standards.

The samples may also be further analyzed for one or more of the following parameters according to standard procedures:
IL-1β, IL-13, TNF-β, total Ig, total IgE, and MCP-1

A reduction of one or more of the above parameters, expect for urea and albumin, is indicative of reduction of inflammation and may constitute an improvement of the COPD condition.

### Serum Analysis

Serum samples may be collected and analysed for the following inflammatory mediators according to standard procedures:
IL-4, IL-6, IL-8, TNF-α and GRO-α

The samples may also be further analyzed for one or more of the following parameters according to standard procedures:
IL-1β, IL-13, TNF-β, total Ig, total IgE, and MCP-1

A reduction of one or more of the above parameters is indicative of reduction of inflammation and may constitute an improvement of the COPD condition.

### Forced Expiratory Volume (FEV₁) to Forced Vital Capacity (FVC) FEV₁/FVC) and Forced Expiratory Volume (FEV₁)

After pre-medication with 1.5 mg of terbutaline, administered in 3 doses via turbohaler FEV₁, FVC and FEV₁/FVC assessment is performed by spirometry according to ERS/ATS standards.

A increase of the FEV₁ value and FEV₁/FVC ratio or a reduction or halt of the progressing decrease of the FEV₁ value and FEV₁/FVC ratio is indicative of an improvement of the COPD condition.

### Number and/or severity of exacerbations

Exacerbations are defined as a sustained worsening of symptoms acute in onset, such as worsening breathlessness, cough, increased sputum production and change in sputum color which necessitates a visit to doctor, emergency department or hospitalization and/or steroid tablets and/or antibiotics.

The number of exacerbations are measured as number of antibiotic regimens, number of steroid tablets regimes, number of visits to/from a doctor, emergency department visits or hospitalizations over a period, e.g. over a 12 month period. A decrease in the number and/or severity of exacerbations is indicative of an improvement of the COPD condition.

### Bronchoalveolar lavage

A bronchoalveolar lavage (BAL) is performed before bronchial biopsies are taken. The scope is advanced until in "clivage" in the right middle lope. 50 ml of isotonic saline is installed and suction is applied. A total of 100 ml is used. At least 30 ml should be harvested. Otherwise, another portion of 50 ml should be installed. The following inflammatory mediators are analysed by standard procedures:

The samples may also be further analyzed for one or more of the following parameters according to standard procedures:
IL-4, IL-6, IL-8, TNF-α, GRO-α, urea and albumin

Urea and albumin are included as reference standards.

The samples may also be further analyzed for one or more of the following parameters according to standard procedures:
IL-1β, IL-13, TNF-β, total Ig, total IgE, and MCP-1

A reduction of one or more of the above parameters, expect for urea and albumin, is indicative of reduction of inflammation and may constitute an improvement of the COPD condition.

### Bronchial biopsy

Five (5) biopsies are obtained from the right or left side (sub-carina between right upper lope and intermediar bronchus, sub-carina at entrance to middle lope (right lung), sub-carina between 8 and 9 or 9-10 lower lope bronchus). The biopsies should be obtained from the same locations when comparing the expression before and following treatment with a CD20 binding full-length antibody or bivalent fragment thereof comprising two Fab fragments linked by a disulfide bridge at the hinge region.

The following parameters are analysed by standard procedures, including Hematoxylin and eosin stain (HE-stain):
CD45+, CD3+, CD4+, CD8+, CD19+, CD20+, CD68+, elastase, EG2 (eosinophils) and AA1 (mast cells)

A reduction of one or more of the above parameters is indicative of an improvement of the COPD condition.

### Medical Research Council (MRC) scale

The Medical Research Council (MRC) dyspnea scale is used for grading the effect of breathlessness on daily activities, cf. Mahler DA et al. (1987) Am. Rev. Respir. Dis. 135:1229-1233; Mahler DA et al. (1988) Chest 93:580-586; and Eltayara LMR et al. (1996) Am. J. Respir. Crit. Care Med. 154:1726-1734. This scale actually measures perceived respiratory disability, the WHO definition of disability being "any restriction or lack of ability to perform an activity in the manner or within the range considered normal for a human being".

A clinically meaningful reduction of dyspnea is indicative of an improvement of the COPD condition.

Baseline Dyspnea Index (BDI) and Transitional Dyspnea Index (TDI)

The Baseline Dyspnea Index (BDI) and Transitional Dyspnea Index (TDI) may be used for evaluating dyspnea, cf. Mahler DA et al. (1984) Chest 85:751-7588.

A clinically meaningful reduction of dyspnea is indicative of an improvement of the COPD condition.

### Example 6: Analysis of cytokines, ureum and album in in BAL samples

Bronchial alveolar lavage (BAL) was performed in a COPD patient included in a clinical trial, at 3 weeks before and 12 weeks after start of treatment with ofatumumab according to the following treatment regimen: at day 0, 100 mg of ofatumumab was administered; at day 7, 100 mg of ofatumumab was administered; and at day 21, 1000 mg of ofatumumab was administered. BAL samples were stored at -20°C. Upon analysis the BAL samples were thawed at room temperature and analysed for the presence of IL-6, IL-8, TNF-α and GRO-α by cytokine specific ELISA as described by the manufacturer (Quantikine human IL-6 immunoassay, cat no: HS600B; Quantikine human CXCL8/IL-8 immunoassay, D8000C; Quantikine human TNFα/TNFSF1A, HSTA00C; Quantikine human CXCL1/GRO-α immunoassay, DRG00; R&D systems, Minneapolis, USA). Urea levels were determined in a colorimetric assay format as described by the manufacturer (Quantichrom Urea assay kit, cat No DIUR-500, Bioassay systems, Hayward CA, USA). Albumin levels were detected by ELISA (Albumin ELISA kit, K6330, Immunodiagnostics, Bensheim, Germany). Optical density levels were detected using an ELISA-reader and concentrations of specific proteins were calculated based on the standard curves provided with the kits. Table 1 shows that 12 weeks after start of treatment reduced levels of IL-6, IL-8 and GRO-α were detected in the BAL samples compared to levels before treatment of the patient. No detectable TNF-α levels were found before or after treatment. Urea levels and albumin levels in the BAL fluid were comparable before and after HuMax-CD20 treatment.

**Table 1 : Cytokine, urea and albumin concentrations in BAL fluid before and after ofatumumab treatment**

| **Sample** | **Time of BAL withdrawal** | **Concentration of undiluted sample (pg/ mL)** | | | | **mg/ dL urea** | **mg/ L albumin** |
|---|---|---|---|---|---|---|---|
| | | IL-6 | IL-8 | TNF-α | GRO-α | | |
| 0701-37076 | Week -3 | 8.96 | 1569.07 | < 0.12 | 1986.35 | 0.16 | 29.61 |
| 0701-37077 | Week +12 | 1.49 | 6.57 | < 0.12 | 929.60 | 0.18 | 25.29 |

### COMBINATIONS

Any combination of the embodiments disclosed in the dependent claims are also contemplated to be within the scope of the invention.

## Claims

1. Use of a CD20 binding full-length antibody or bivalent fragment thereof comprising two Fab fragments linked by a disulfide bridge at the hinge region for the preparation of a medicament for the treatment of Chronic Obstructive Pulmonary Disease (COPD).

2. Use according to claim 1, **characterized in that** the antibody is a monoclonal antibody against CD20.

3. Use according to claim 2, **characterized in that** the antibody is a human monoclonal antibody against CD20.

4. Use according to any one of the claims 2-3, **characterized in that** the antibody is a full-length IgG1 antibody.

5. Use according to any one of the claims 2-4, **characterized in that** the antibody against CD20 binds to mutant P172S CD20 (proline at position 172 mutated to serine) with at least the same affinity as to human CD20.

6. Use according to any one of the claims 2-5, **characterized in that** the antibody against CD20 binds to an epitope on CD20
(i) which does not comprise or require the amino acid residue proline at position 172;
(ii) which does not comprise or require the amino acid residues alanine at position 170 or proline at position 172;
(iii) which comprises or requires the amino acid residues asparagine at position 163 and asparagine at position 166;
(iv) which does not comprise or require the amino acid residue proline at position 172, but which comprises or requires the amino acid residues asparagine at position 163 and asparagine at position 166; or
(v) which does not comprise or require the amino acid residues alanine at position 170 or proline at position 172, but which comprises or requires the amino acid residues asparagine at position 163 and asparagine at position 166.

7. Use according to any one of the claims 2-6, **characterized in that** the antibody against CD20 comprises a V_{H} CDR1 of SEQ ID NO:3, a V_{H} CDR2 of SEQ ID NO:4, a V_{H} CDR3 of SEQ ID NO:5, a V_{L} CDR1 of SEQ ID NO:6, a V_{L} CDR2 of SEQ ID NO:7 and a V_{L} CDR3 sequence of SEQ ID NO:8.

8. Use according to any one of the claims 2-6, **characterized in that** the antibody against CD20 has human heavy chain and human light chain variable regions comprising the amino acid sequences as set forth in SEQ ID NO:1 and SEQ ID NO:2, respectively; or amino acid sequences which are at least 95% homologous, and more preferably at least 98%, or at least 99% homologous to the amino acid sequences as set forth in SEQ ID NO:1 and SEQ ID NO:2, respectively.

9. Use according to claim 1, **characterized in that** the CD20 binding antibody is selected from ofatumumab (2F2), 11B8, 7D8, 2C6, AME-133, TRU-015, IMMU-106, ocrelizumab (2H7.v16, PRO-70769, R-1594), tositumomab and rituximab.

10. Use according to any one of the preceding claims, wherein the medicament is suitable for administration in combination with one or more further therapeutic agents.

11. A CD20 binding full-length antibody or bivalent fragment thereof comprising two Fab fragments linked by a disulfide bridge at the hinge region for use in the treatment of Chronic Obstructive Pulmonary Disease (COPD).

12. A CD20 binding antibody for use according to claim 11, wherein the antibody is as defined in any one of claims 2-9.

13. A pharmaceutical composition for use in the treatment of Chronic Obstructive Pulmonary Disease (COPD), **characterized in that** it contains a CD20 binding full-length antibody or bivalent fragment thereof comprising two Fab fragments linked by a disulfide bridge at the hinge region.

14. A pharmaceutical composition for use according to claim 13, wherein the antibody is as defined in any one of claims 2-9.

## Patentansprüche

1. Verwendung eines CD20-bindenden Volllängen-Antikörpers oder bivalenten Fragments davon, umfassend zwei Fab-Fragmente, die durch eine Disulfidbrücke an der Hinge-Region verknüpft sind, für die Herstellung eines Medikaments zur Behandlung von chronisch-obstruktiver Lungenerkrankung (COPD).

2. Verwendung gemäß Anspruch 1, **dadurch gekennzeichnet, dass** der Antikörper ein monoklonaler Antikörper gegen CD20 ist.

3. Verwendung gemäß Anspruch 2, **dadurch gekennzeichnet, dass** der Antikörper ein humaner monoklonaler Antikörper gegen CD20 ist.

4. Verwendung gemäß einem der Ansprüche 2 bis 3, **dadurch gekennzeichnet, dass** der Antikörper ein Volllängen-IgG1-Antikörper ist.

5. Verwendung gemäß einem der Ansprüche 2 bis 4, **dadurch gekennzeichnet, dass** der Antikörper gegen CD20 an mutantes P172S-CD20 (Prolin an Position 172 mutiert zu Serin) mit wenigstens derselben Affinität wie an humanes CD20 bindet.

6. Verwendung gemäß einem der Ansprüche 2 bis 5, **dadurch gekennzeichnet, dass** der Antikörper gegen CD20 an ein Epitop an CD20 bindet,
(i) das den Aminosäurerest Prolin an Position 172 nicht umfasst oder benötigt;
(ii) das die Aminosäurereste Alanin an Position 170 oder Prolin an Position 172 nicht umfasst oder benötigt;
(iii) das die Aminosäurereste Asparagin an Position 163 und Asparagin an Position 166 umfasst oder benötigt;
(iv) das den Aminosäurerest Prolin an Position 172 nicht umfasst oder benötigt, aber die Aminosäurereste Asparagin an Position 163 und Asparagin an Position 166 umfasst oder benötigt, oder
(v) das die Aminosäurereste Alanin an Position 170 oder Prolin an Position 172 nicht umfasst oder benötigt, aber die Aminosäurereste Asparagin an Position 163 und Asparagin an Position 166 umfasst oder benötigt.

7. Verwendung gemäß einem der Ansprüche 2 bis 6, **dadurch gekennzeichnet, dass** der Antikörper gegen CD20 eine V_{H}-CDR1 mit SEQ ID NO:3, eine V_{H}-CDR2 mit SEQ ID NO:4, eine V_{H}-CDR3 mit SEQ ID NO:5, eine V_{L}-CDR1 mit SEQ ID NO:6, eine V_{L}-CDR2 mit SEQ ID NO:7 und eine V_{L}-CDR3-Sequenz mit SEQ ID NO:8 umfasst.

8. Verwendung gemäß einem der Ansprüche 2 bis 6, **dadurch gekennzeichnet, dass** der Antikörper gegen CD20 variable Regionen der humanen schweren Kette und der humanen leichten Kette hat, die die Aminosäuresequenzen, wie sie in SEQ ID NO:1 bzw. SEQ ID NO:2 angegeben sind, oder Aminosäuresequenzen umfassen, die wenigstens 95% homolog und bevorzugter wenigstens 98% oder wenigstens 99% homolog zu den in SEQ ID NO:1 bzw. SEQ ID NO:2 angegebenen Aminosäuresequenzen sind.

9. Verwendung gemäß Anspruch 1, **dadurch gekennzeichnet, dass** der CD20-bindende Antikörper aus Ofatumumab (2F2), 11B8, 7D8, 2C6, AME-133, TRU-015, IMMU-106, Ocrelizumab (2H7.v16, PRO-70769, R-1594), Tositumomab und Rituximab ausgewählt ist.

10. Verwendung gemäß einem der vorangehenden Ansprüche, wobei das Medikament zur Verabreichung in Kombination mit einem oder mehreren weiteren therapeutischen Mittel(n) geeignet ist.

11. CD20-bindender(s) Volllängen-Antikörper oder bivalentes Fragment davon, umfassend zwei Fab-Fragmente, die durch eine Disulfidbrücke in der Hinge-Region verknüpft sind, zur Verwendung bei der Behandlung von chronisch-obstruktiver Lungenerkrankung (COPD).

12. CD20-bindender Antikörper zur Verwendung gemäß Anspruch 11, wobei der Antikörper wie in einem der Ansprüche 2-9 definiert ist.

13. Pharmazeutische Zusammensetzung zur Verwendung bei der Behandlung von chronisch-obstruktiver Lungenerkrankung (COPD), **dadurch gekennzeichnet, dass** sie ein(en) CD20-bindenden(s) Volllängen-Antikörper oder bivalentes Fragment davon, umfassend zwei Fab-Fragmente, die durch eine Disulfidbrücke in der Hinge-Region verknüpft sind, enthält.

14. Pharmazeutische Zusammensetzung zur Verwendung gemäß Anspruch 13, wobei der Antikörper wie in einem der Ansprüche 2-9 definiert ist.

## Revendications

1. Utilisation d'un anticorps complet se liant à CD20, ou d'un fragment bivalent d'un tel anticorps comprenant deux fragments Fab raccordés par un pont disulfure au niveau de la région charnière, pour la préparation d'un médicament conçu pour le traitement de la broncho-pneumopathie chronique obstructive (BPCO).

2. Utilisation conforme à la revendication 1, **caractérisée en ce que** l'anticorps est un anticorps monoclonal dirigé contre CD20.

3. Utilisation conforme à la revendication 2, **caractérisée en ce que** l'anticorps est un anticorps monoclonal humain dirigé contre CD20.

4. Utilisation conforme à l'une des revendications 2 et 3, **caractérisée en ce que** l'anticorps est un anticorps IgG1 complet.

5. Utilisation conforme à l'une des revendications 2 à 4, **caractérisée en ce que** l'anticorps dirigé contre CD20 se lie au CD20 mutant P172S (le résidu de proline en position 172 est remplacé par un résidu de sérine) avec au moins la même affinité qu'au CD20 humain.

6. Utilisation conforme à l'une des revendications 2 à 5, **caractérisée en ce que** l'anticorps dirigé contre CD20 se lie à un épitope de CD20
i) qui ne comporte pas ou ne nécessite pas la présence d'un résidu d'acide aminé proline en position 172 ;
ii) qui ne comporte pas ou ne nécessite pas la présence d'un résidu d'acide aminé alanine en position 170 ou proline en position 172 ;
iii) qui comporte ou nécessite la présence de résidus d'acide aminé asparagine en position 163 et asparagine en position 166 ;
iv) qui ne comporte pas ou ne nécessite pas la présence d'un résidu d'acide aminé proline en position 172, mais comporte ou nécessite la présence de résidus d'acide aminé asparagine en position 163 et asparagine en position 166 ;
v) ou qui ne comporte pas ou ne nécessite pas la présence d'un résidu d'acide aminé alanine en position 170 ou proline en position 172, mais comporte ou nécessite la présence de résidus d'acide aminé asparagine en position 163 et asparagine en position 166.

7. Utilisation conforme à l'une des revendications 2 à 6, **caractérisée en ce que** l'anticorps dirigé contre CD20 comprend une région CDR1 de région V_{H} de Séquence N° 3, une région CDR2 de région V_{H} de Séquence N° 4, une région CDR3 de région V_{H} de Séquence N° 5, une région CDR1 de région V_{L} de Séquence N° 6, une région CDR2 de région V_{L} de Séquence N° 7, et une région CDR3 de région V_{L} de Séquence N° 8.

8. Utilisation conforme à l'une des revendications 2 à 6, **caractérisée en ce que** l'anticorps dirigé contre CD20 comporte des régions variables de chaîne lourde humaine et de chaîne légère humaine comprenant les séquences d'acides aminés données respectivement en tant que Séquence N° 1 et Séquence N° 2, ou des séquences d'acides aminés qui sont homologues, à un degré d'au moins 95 %, de préférence d'au moins 98 %, ou d'au moins 99 %, aux séquences d'acides aminés données respectivement en tant que Séquence N° 1 et Séquence N° 2.

9. Utilisation conforme à la revendication 1, **caractérisée en ce que** l'anticorps se liant à CD20 est choisi parmi les suivants : ofatumumab (2F2), 11B8, 7D8, 2C6, AME-133, TRU-015, IMMU-106, ocrelizumab (2H7.v16, PRO-70769, R-1594), tositumomab et rituximab.

10. Utilisation conforme à l'une des revendications précédentes, dans laquelle le médicament est approprié pour être administré en combinaison avec un ou plusieurs autre(s) agent(s) thérapeutique(s).

11. Anticorps complet se liant à CD20, ou fragment bivalent d'un tel anticorps comprenant deux fragments Fab raccordés par un pont disulfure au niveau de la région charnière, pour utilisation dans le traitement de la broncho-pneumopathie chronique obstructive (BPCO).

12. Anticorps se liant à CD20 pour utilisation conforme à la revendication 11, lequel anticorps est tel que défini dans l'une des revendications 2 à 9.

13. Composition pharmaceutique pour utilisation dans le traitement de la broncho-pneumopathie chronique obstructive (BPCO), **caractérisée en ce qu'**elle contient un anticorps complet se liant à CD20, ou un fragment bivalent d'un tel anticorps comprenant deux fragments Fab raccordés par un pont disulfure au niveau de la région charnière.

14. Composition pharmaceutique pour utilisation conforme à la revendication 13, dans laquelle l'anticorps est tel que défini dans l'une des revendications 2 à 9.
